# EUROPEAN PATENT APPLICATION

(11) **EP 2 746 253 A1**
(43) Date of publication of application: **25.06.2014**
(21) Application number: 12199046.9
(22) Date of filing: 21.12.2012
(51) Int. Cl.: C07D 209/88, C07D 213/53, C07D 215/12, C07D 307/81, C07D 333/58, A61Q 19/02

(54) **Novel compounds with skin lightening properties**

(71) Applicant: Unilever PLC, London, Greater London EC4Y 0DY (GB)
(72) Inventor: The designation of the inventor has not yet been filed
(74) Representative: Acham, Nicholas Clive

(57) **Abstract**

This invention relates to novel compounds with skin lightening properties based on an aromatic hydrazine carboxyimidoamide structure, topical skin lightening compositions comprising the same and a method of skin lightening using the said compositions. In particular, the skin lightening benefit is selected from any one of the group consisting of treating melasma, treating post-inflammatory hyperpigmentation, treating age spots, treating dark spots and treating uneven skin tone.

Desired skin colour is a major unmet consumer need around the world and especially in Asia. Consumers particularly desire even skin colour, absence of age spots (solar lentigines), absence of hyperpigmentation and lighter overall skin tone. One solution is to use biological actives that reduce the activity of melanocyte cells in skin. These cells, present in the basal layer of the epidermis, make the dark coloured pigment melanin and export it, in small export vesicles called melanosomes, to the neighbouring keratinocytes. It is well described in the literature that compounds which reduce melanin synthesis when topically applied to the skin will reduce skin darkness over time and can generate a more even skin tone.

Tyrosinase is a very popular target for the regulation of melanocyte pigment production. However effective inhibitors of tyrosinase are bedevilled by safety issues causing, for example, melanocyte cell death, permanent depigmentation, irritation and allergic reactions. Often effective inhibitors kill melanocytes (for example hydroquinone) or cause sensitisation reactions. There is therefore a great need for safe and effective inhibitors of skin pigment production that work through an alternative safe mechanism.

The inventors have observed that selected compounds of the same generic structure inhibit the production of soluble melanin by B16 mouse melanoma cells without cell toxicity.

## Description

This invention relates to novel compounds with skin lightening properties based on an aromatic hydrazine carboxyimidoamide structure, topical skin lightening compositions comprising the same and a method of skin lightening using the said compositions. In particular, the skin lightening benefit is selected from any one of the group consisting of treating melasma, treating post-inflammatory hyperpigmentation, treating age spots, treating dark spots and treating uneven skin tone.

Desired skin colour is a major unmet consumer need around the world and especially in Asia. Consumers particularly desire even skin colour, absence of age spots (solar lentigines), absence of hyperpigmentation and lighter overall skin tone. One solution is to use biological actives that reduce the activity of melanocyte cells in skin. These cells, present in the basal layer of the epidermis, make the dark coloured pigment melanin and export it, in small export vesicles called melanosomes, to the neighbouring keratinocytes. It is well described in the literature that compounds which reduce melanin synthesis when topically applied to the skin will reduce skin darkness over time and can generate a more even skin tone.

Tyrosinase is a very popular target for the regulation of melanocyte pigment production. However effective inhibitors of tyrosinase are bedevilled by safety issues causing, for example, melanocyte cell death, permanent depigmentation, irritation and allergic reactions. Often effective inhibitors kill melanocytes (for example hydroquinone) or cause sensitisation reactions. There is therefore a great need for safe and effective inhibitors of skin pigment production that work through an alternative safe mechanism.

The inventors have observed that selected compounds of the same generic structure inhibit the production of soluble melanin by B16 mouse melanoma cells without cell toxicity. B16 mouse melanoma cells are a cell line derived from a mouse melanoma that secret soluble melanin into the culture medium, facilitating assays that measure regulation of pigment production. Compounds not falling within the aforementioned generic structure did not reduce melanin production by the B16 mouse melanoma cells. Select materials positive in the B16 mouse melanoma assay were also tested in human cell living skin equivalents containing keratinocytes and melanocytes (so called Melanoderms^{™}). Melanoderms^{™} are in vitro 3D living skin equivalents containing both human keratinocytes and melanocytes and are used as a close mimic of the effect of compounds on human skin function in vivo. Compounds falling within the aforementioned generic structure were found to inhibit melanin production measured using living skin equivalents without toxicity.

Accordingly the aforementioned generic structure describes compounds which inhibit pigment production and which can be used to beneficially alter skin colour and treat hyperpigmentation disorders in a safe and effective manner.

The selected compounds are novel and hence are not documented in the commercial database SciFinder (RTM) and accordingly do not have Chemical Abstracts Service (CAS) numbers.

### Summary of the Invention

In a first aspect of the invention, a compound of structure I: 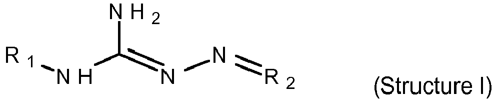 or a salt or a stereoisomer or a tautomer thereof is provided, wherein R₁ is 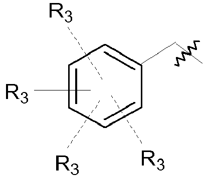 wherein R₃ = F, Cl, OCH₃, COOCH₃, OH or Me; wherein if R₂ is a benzofuran moiety or 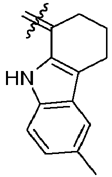 R₁ can be a phenyl group;
wherein R₂ is 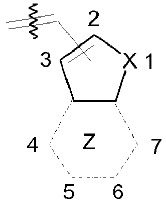 connected via either one of the two carbon atoms numbered 2 or 3; wherein X1 = O or S; wherein if Z is not present, the heterocycle containing X1 contains another double bond; wherein Z is a six membered aromatic or non-aromatic heterocyclic, or cycloalkyl or aryl moity, optionally substituted at one or more available carbons numbered 4 to 7 with R₃;
or R₂ is 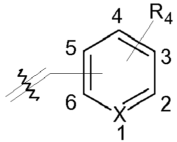 connected via either one of the two carbon atoms numbered 5 or 6; wherein X1 = C or N; wherein R₄ is a 6 membered pendant monoheterocyclic ring attached at any available carbon, or a 6 membered fused aromatic ring, fused at any available pair of carbons;
or R₂ is

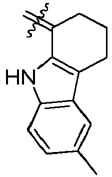

In a second aspect of the invention, a topical skin lightening composition is provided, the topical skin lightening composition comprising one or more compounds of the first aspect of the invention and a dermatologically acceptable carrier.

In a third aspect of the invention, a method of skin lightening is provided, the method of skin lightening comprising the step of applying to skin the topical skin lightening composition of the second aspect of the invention.

### Detailed Description of the Invention

In a first aspect of the invention, a compound of structure I: 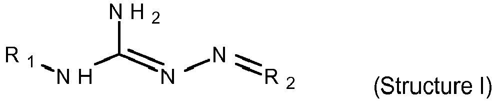 or a salt or a stereoisomer or a tautomer thereof is provided.

In the skin lightening compound of structure I, Z may optionally not be substituted at one or more available carbons numbered 4 to 7 with R₃. Optionally Z is aromatic.

In the skin lightening compound of structure I, R₄ may optionally be an aromatic ring.

In the skin lightening compound of structure I, R₁ may optionally be singly or doubly substituted by R₃.

R₂ may be preferably a benzothiophene or a benzofuran moiety. In the alternative, R₂ may be preferably a quinoline moiety.

The skin lightening compound of the first aspect of the invention may be a salt of structure (I). The counterion may be selected from the group consisting of phosphate, hydrogen monophosphate, hydrogen biphosphate, carbonate, bicarbonate, formate, acetate, nitrate, sulphate, hydrogen sulphate, sulphite, metabisulphite, hydroxide, fluoride, chloride, bromide, iodide, lactate, salicyclate, phytate, lipoate, tannate, gallate, a fatty acid anion and an organic fruit acid anion.

Whilst the fatty acid anion may be any fatty acid anion suitable for use in topical compositions, preferably the fatty acid anion is stearate or palmitate.

The organic fruit acid anion is preferably selected from the group consisting of malate, quinate, alpha ketoglutarate, oxalacetate, citrate, pyruvate, fumarate, lactate, succinate, tartrate, acetate, formate, isocitrate, lactoisocitrate, shikimate, oxalate, glycerate citramalate, glycolate, glucuronate, galacturonate, glutamate, aspartate, benzoate, ascorbate, salicylate and sorbate.

The skin lightening compound of the first aspect of the invention may be in the form of a stereoisomer of structure I, in particular an E stereoisomer or a Z stereoisomer. The skin lightening compound of the first aspect of the invention may also be a structural isomer of structure I, in particular it may be a tautomer of structure I including: 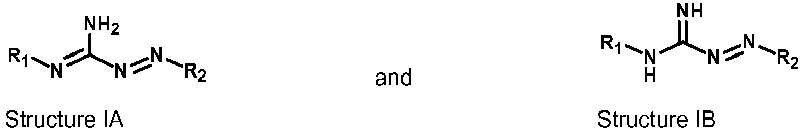 whilst the three tautomers can be generically represented by the following structure:

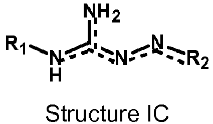

The skin lightening compound of the first aspect of the invention may, in particular, be selected from the group consisting of (E)-1-amino-N-(4-hydroxybenzyl)-1-{(2E)-[4-(pyridin-4-yl)benzylidene]hydrazinylidenelmethanamine (NC-0498), (E)-1-amino-1-[(2E)-(1-benzothiophen-3-ylmethylidene)hydrazinylidene]-N-[4-(methoxycarbonyl)benzyl] methanamine (NC-051 0), (E)-1-amino-N-[4-(methoxycarbonyl)benzyl]-1-[(2E)-(quinolin-2-ylmethylidene)hydrazinylidene] methanamine (NC-511), (E)-1-amino-1-[(2E)-(1-benzothiophen-3-ylmethylidene)hydrazinylidene]-N-(3,4-dichlorobenzyl) methanamine (NC-0515), (E)-1-amino-1-[(2E)-(1-benzothiophen-3-ylmethylidene)hydrazinylidene]-N-(4-fluorobenzyl) methanamine (NC-0517), (E)-1-amino-1-[(2E)-(1-benzofuran-2-ylmethylidene)hydrazinylidene]-N-benzylmethanaminium iodide (NC-519), (E)-1-amino-1-[(2E)-(1-benzofuran-2-ylmethylidene)hydrazinylidene]-N-[4-(methoxycarbonyl)benzyl] methanamine (NC-0521), (E)-1-amino-1-[(2E)-(1-benzothiophen-2-ylmethylidene)hydrazinylidene]-N-[4-(methoxycarbonyl)benzyl] methanamine (NC-0522), (E)-1-amino-1-[(2E)-(1-benzothiophen-3-ylmethylidene)hydrazinylidene]-N-[3-(methoxycarbonyl)benzyl] methanamine (NC-0525), (E)-1-amino-1-[(2E)-(1-benzothiophen-3-ylmethylidene)hydrazinylidene]-N-(4-methoxybenzyl)methanaminium iodide (NC-0528), (E)-1-amino-1-[(2E)-(1-benzofuran-2-ylmethylidene)hydrazinylidene]-N-(4-methoxybenzyl) methanamine (NC-0531), (E)-1-amino-N-(4-fluorobenzyl)-1-{(2Z)-[6-methyl-2,3,4,9-tetrahydro-1H-carbazol-1-ylidene]hydrazinylidene} methanamine (NC-0536), (E)-1-amino-N-(2,6-dichlorobenzyl)-1-{(2Z)-[6-methyl-2,3,4,9-tetrahydro-1H-carbazol-1-ylidene]hydrazinylidene} methanamine (NC-0537), or a salt or stereoisomer or tautomer thereof.

Preferably the skin lightening compound of the first aspect of the invention is selected from the group consisting of (E)-1-amino-1-[(2E)-(1-benzothiophen-3-ylmethylidene)hydrazinylidene]-N-[4-(methoxycarbonyl)benzyl] methanamine (NC-0510), (E)-1-amino-N-[4-(methoxycarbonyl)benzyl]-1-[(2E)-(quinolin-2-ylmethylidene)hydrazinylidene] methanamine (NC-0511), (E)-1-amino-1-[(2E)-(1-benzothiophen-3-ylmethylidene)hydrazinylidene]-N-(4-fluorobenzyl)methanaminium iodide (NC-0517), (E)-1-amino-1-[(2E)-(1-benzofuran-2-ylmethylidene)hydrazinylidene]-N-[4-(methoxycarbonyl)benzyl] methanamine (NC-521), (E)-1-amino-1-[(2E)-(1-benzothiophen-2-ylmethylidene)hydrazinylidene]-N-[4-(methoxycarbonyl)benzyl] methanamine (NC-0522), (E)-1-amino-1-[(2E)-(1-benzofuran-2-ylmethylidene)hydrazinylidene]-N-(4-methoxybenzyl) methanamine (NC-0531), or a salt or stereoisomer or tautomer thereof.

Most preferably the skin lightening compound of the first aspect of the invention is selected from the group consisting of (E)-1-amino-N-[4-(methoxycarbonyl)benzyl]-1-[(2E)-(quinolin-2-ylmethylidene)hydrazinylidene] methanamine (NC-0511), (E)-1-amino-1-[(2E)-(1-benzofuran-2-ylmethylidene)hydrazinylidene]-N-[4-(methoxycarbonyl)benzyl] methanamine (NC-0521), (E)-1-amino-1-[(2E)-(1-benzothiophen-2-ylmethylidene)hydrazinylidene]-N-[4-(methoxycarbonyl)benzyl] methanamine (NC-0522), or a salt or stereoisomer or tautomer thereof.

In a second aspect of the invention, a topical skin lightening composition is provided, the topical skin lightening composition comprising one or more compounds of the first aspect of the invention and a dermatologically acceptable carrier.

Typically, the topical skin lightening composition comprises 0.001-5, preferably 0.01-2, most preferably 0.1-1 % w/w of one or more compounds of the first aspect of the invention.

The topical skin lightening composition may additionally comprise at least one additional skin lightening compound not falling within structure (I), or a salt or a stereoisomer thereof. Examples include a compound selected from the group consisting of niacinamide, a resorcinol, koijic acid, retinol, retinyl esters including retinyl palmitate, 12-hydroxystearic acid, lactic acid, ascorbic acid, glabradin, dioic acids including octadecenedioic acid and azeleic acid, resveratrol, ascorbyl phosphate, ascorbyl palmitate, acetyl glucosamine, calcium pantothenate, alpha arbutin, climbazole, pitera extract, soybean extract, undecylenoyl phenylalanine and aqueous extract of bearberry. The resorcinol may be selected from the group consisting of 4-ethyl resorcinol, 4-hexyl resorcinol and phenylethyl resorcinol.

Typically, the topical skin lightening composition comprises 0.0001-10, preferably 0.01-2, most preferably 0.1-1 % w/w of the at least one additional skin lightening compound.

The topical skin lightening composition of the second aspect of the invention preferably comprises a sunscreen to protect the skin against UV-A and/or UV-B solar radiation. Sunscreens include those materials commonly employed to block ultraviolet light. Illustrative organic compounds are the derivatives of p-aminobenzoic acid (PABA), cinnamate and salicylate. For example, avobenzophenone (Parsol 1789®), octyl methoxycinnamate and 2-hydroxy-4-methoxy benzophenone (also known as oxybenzone) can be used. Octyl methoxycinnamate and 2-hydroxy-4-methoxy benzophenone are commercially available under the trade marks, Parsol MCX and Benzophenone-3, respectively. Ecamsule, a benzylidene camphor derivative, sold under the trade mark Mexoryl SX, and drometrizole trisiloxane, a benzotriazole sold under the trade mark Mexoryl XL, may also be used. Still other examples include octocrylene, phenylbenzimidazole sulfonic acid (also known as ensulizole), ethylhexyl salicylate,diethylhexyl naphthylate, bimotrizinole (trade marked as Tinosorb S) and bisoctrizole (Tinosorb M).

Inorganic sunscreens include oxides like titanium dioxide and zinc oxide which reflect or scatter the suns rays. The exact amount of sunscreen employed in the compositions can vary depending upon the degree of protection desired from the sun's UV radiation. However levels can be in the range 0.01 to 15, preferably 0.1 to 10, most preferably 0.5 to 7.5 % by weight of the topical skin lightening composition of the second aspect of the invention, including all ranges subsumed therein.

The dermatologically acceptable carrier for the topical skin lightening composition of the second aspect of the invention act as diluents, dispersants and/or carriers for the compound of the first aspect of the invention and for any other optional but often preferred ingredients. The carrier may be aqueous-based, anhydrous or an emulsion whereby a water-in-oil or oil-in-water emulsion is generally preferred. If the use of water is desired, water typically makes up the balance of the topical skin lightening composition of the second aspect of the invention, and preferably makes up from 5 to 98 %, and most preferably from 40 to 80 % by weight of the topical skin lightening composition of the second aspect of the invention, including all ranges subsumed therein.

In addition to water, organic solvents may be optionally included to act as carriers or to assist carriers within the compositions of the present invention. Illustrative and non-limiting examples of the types of organic solvents suitable for use in the present invention include alkanols like ethyl and isopropyl alcohol, mixtures thereof or the like.

Other optional additives suitable for use include ester oils like isopropyl myristate, cetyl myristate, 2-octyldodecyl myristate, avocado oil, almond oil, olive oil, neopentylglycol dicaprate, mixtures thereof or the like. Typically, such ester oils assist in emulsifying the composition of this invention, and an effective amount is often used to yield a stable, and most preferably, water-in-oil emulsion.

Emollients may also be used, if desired, as carriers within the composition of the present invention. Alcohols like 1-hexadecanol (i.e. cetyl alcohol) are often desired as are the emollients generally classified as silicone oils and synthetic esters. Silicone oils suitable for use include cyclic or linear polydimethylsiloxanes containing from 3 to 9, preferably from 4 to 5, silicon atoms. Non-volatile silicone oils useful as an emollient material in the inventive composition described herein include polyalkyl siloxanes, polyalkylaryl siloxanes and polyether siloxane copolymers. The essentially non-volatile polyalkyl siloxanes useful herein include, for example, polydimethylsiloxanes. Silicone elastomers may also be used.

The ester emollients that may optionally be used are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, stearyl stearate and arachidyl behenate.
(5) Sterols esters, of which cholesterol fatty acid esters are examples.

Emollients, when used, typically make up from 0.1 to 50 % by weight of the topical skin lightening composition of the second aspect of the invention, including all ranges subsumed therein.

Fatty acids having from 10 to 30 carbon atoms may also be included as acceptable carriers within the composition of the present invention. Illustrative examples of such fatty acids include pelargonic, lauric, myristic, palmitic, stearic, isostearic, oleic, linoleic, arachidic, behenic or erucic acid, and mixtures thereof. Compounds that are believed to enhance skin penetration, like dimethyl sulfoxide, fatty acids and ethanol may also be used as an optional carrier.

Humectants of the polyhydric alcohol type may also be employed in the compositions of this invention. The humectant often aids in increasing the effectiveness of the emollient, reduces scaling at the skin surface, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. Other humectants which may be used include hydroxyethyl urea. The amount of humectant may range anywhere from 0.2 to 25%, and preferably, from 0.5 to 15 % by weight of the topical skin lightening composition of the second aspect of the invention, including all ranges subsumed therein.

Moisturisation may be improved through use of petrolatum or paraffins.

Thickeners may also be utilized as part of the acceptable carrier in the compositions of the present invention. Typical thickeners include cross-linked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0 to 5, usually from 0.001 to 1, optimally from 0.01 to 0.5 % by weight of the topical skin lightening composition of the second aspect of the invention, including all ranges subsumed therein.

Collectively the water, solvents, silicones, esters, fatty acids, humectants and/or thickeners will constitute the acceptable carrier in amounts from 1 to 99.9, preferably from 80 to 99 % by weight of the topical skin lightening composition of the second aspect of the invention, including all ranges subsumed therein.

Surfactants may also be present in compositions of the present invention. Total concentration of the surfactant will range from about 0 to about 40%, and preferably from about 0 to about 20%, optimally from about 0 to about 5% by weight of the composition. The surfactant may be selected from the group consisting of anionic, nonionic, cationic and amphoteric actives. Particularly preferred nonionic surfactants are those with a C10-C20 fatty alcohol or acid hydrophobe condensed with from 2 to 100 moles of ethylene oxide or propylene oxide per mole of hydrophobe; mono- and di- fatty acid esters of ethylene glycol; fatty acid monoglyceride; sorbitan, mono- and di- C8-C20 fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C8-C20 acyl isethionates, acyl glutamates, C8-C20 alkyl ether phosphates and combinations thereof.

Perfumes may be used in the composition of this invention. Illustrative non-limiting examples of the types of perfumes that may be used include those comprising terpenes and terpene derivatives like those described in Bauer, K., et al., Common Fragrance and Flavor Materials, VCH Publishers (1990).

Illustrative yet non-limiting examples of the types of fragrances that may be used in this invention include myrcene, dihydromyrenol, citral, tagetone, cis-geranic acid, citronellic acid, mixtures thereof or the like.

Preferably, the amount of fragrance employed in the topical skin lightening composition of the second aspect of the invention is in the range from 0.0 to 10, more preferably 0.00001 to 5, most preferably 0.0001 to 2 % by weight of the topical skin lightening composition of the second aspect of the invention, including all ranges subsumed therein.

Various types of optional additional active ingredients may be used in the compositions of the present invention. Actives are defined as skin benefit agents other than emollients and other than ingredients that merely improve the physical characteristics of the composition. Although not limited to this category, general examples include talcs and silicas, as well as alpha-hydroxy acids, beta-hydroxy acids and zinc salts.

Beta-hydroxy acids include salicylic acid, for example. Zinc oxide and zinc pyrithione are examples of zinc salts useful in the composition of the present invention.

Many compositions, especially those containing water, should be protected against the growth of potentially harmful microorganisms. Anti-microbial compounds, such as triclosan, and preservatives are, therefore, typically necessary. Suitable preservatives include alkyl esters of p-hydroxybenzoic acid, hydantoin derivatives, propionate salts, and a variety of quaternary ammonium compounds. Particularly preferred preservatives of this invention are methyl paraben, propyl paraben, phenoxyethanol and benzyl alcohol. Preservatives will usually be employed in amounts ranging from 0.1 to 2 % by weight of the composition.

Still other optional ingredients that may be used with the topical skin lightening composition of the second aspect of the invention include dioic acids (e.g. malonic acid and sebacic acid), antioxidants like vitamin E, retinoids, including retinoic acid, retinal, retinol and retinyl esters such as retinyl propionate and retinyl palmitate, conjugated linoleic acid, petroselinic acid and mixtures thereof, as well as any other conventional ingredients well known for wrinkle-reducing (such as hyaluronic acid, ubiquinone, jasmonic acid derivatives, collagen, peptides and proxylane), anti-acne effects and reducing the impact of sebum.

The topical skin lightening composition of the second aspect of the invention preferably comprises an antiperspirant active. Antiperspirant actives are preferably incorporated in an amount of from 0.5 to 50, particularly from 5 to 30 and especially from 10 to 26 % by weight of the topical skin lightening composition of the second aspect of the invention, including all ranges subsumed therein. It is often considered that the main benefit from incorporating of up to 5 % by weight of the composition of an antiperspirant active in a stick composition is manifest in reducing body odour, and that as the proportion of antiperspirant active increases, so the efficacy of that composition at controlling perspiration increases.

Antiperspirant actives for use herein are often selected from astringent active salts, including in particular aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates.

Aluminium halohydrates are usually defined by the general formula Al2(OH)xQy.wH20 in which Q represents chlorine, bromine or iodine, x is variable from 2 to 5 and x + y = 6 while wH2O represents a variable amount of hydration. Especially effective aluminium halohydrate salts, known as activated aluminium chlorohydrates, are described in EP 6739 A (Unilever NV et al), the contents of which specification is incorporated herein by reference. Such activated aluminium chlorohydrates are made by a method in which the weight concentration of aluminium compounds in the solution is controlled within specified limits and simultaneously the temperature of that solution is controlled within a specified elevated temperature range whilst polymeric aluminium species are formed, and drying conditions are strictly controlled as described in the said EP 6739 A. Some activated salts do not retain their enhanced activity in the presence of water but are useful in substantially anhydrous formulations, i.e. formulations that do not contain a distinct aqueous phase.

Zirconium actives can usually be represented by the empirical general formula: ZrO(OH)2n-nzBz.wH20 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH20. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have co-ordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH2(NH2)COOH.

It is highly desirable to employ complexes of a combination of aluminium halohydrates and zirconium chlorohydrates together with amino acids such as glycine, which are disclosed in US 3 792 068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis, though with differing particle size distributions.

Many aluminium and/or zirconium-containing astringent antiperspirant salts employed herein have metal: chloride mole ratio in the range of 1.3:1 to 1.5:1. Others having a lower metal:chloride mole ratio, such as from 1:1 to 1.25:1 tend to generate lower pHs when applied to skin and thus tend to be more irritating.

The proportion of solid antiperspirant salt in a suspension composition normally includes the weight of any water of hydration and any complexing agent that may also be present in the solid active.

Many particulate antiperspirants employed in the instant invention have a refractive index (RI) of at least 1.49 and not higher than 1.57. Actives which are free from zirconium tend to have an RI of from 1.49 to 1.54, depending on their formula and at least partly on their residual water content. Likewise, actives which contain zirconium tend to have an RI of from 1.52 to 1.57.

The selection of the antiperspirant active material desirably takes into account the type of applicator from which it is dispensed. Thus, in many particularly preferred embodiments in which the composition is dispensed from a contact applicator, for example using a stick or cream (soft solid dispenser, the antiperspirant active comprises an aluminium-zirconium active, such as AZAG. However, in some other preferred embodiments, the antiperspirant active is highly desirably an aluminium chlorohydrate (ACH) and in others an activated aluminium chlorohydrate (AACH).

For incorporation of compositions according to the present invention, desirably at least 90, preferably at least 95 and especially at least 99 % by weight of the particles have a diameter in the range of from 0.1 up to 100 µm, and usually have an average particle diameter of at least 1 µm and especially below 20 µm. In some highly desirable contact compositions the particles by weight have a weight average particle size of at least 2 µm and particularly below 10 µm, such as in the range of from 3 to 8 µm.

Suitable deodorant actives can comprise deodorant effective concentrations of antiperspirant metal salts, deoperfumes, and/or microbiocides, including particularly bactericides, such as chlorinated aromatics, including biguanide derivatives, of which triclosan (e.g. Irgasan DP300 or Triclorban), and chlorhexidine warrant specific mention. Another class of effective deodorants comprises polyaminopropyl biguanide salts such as are available under the trade mark Cosmosil. Such materials commonly act as bactericides. A still further class of materials that can inhibit malodour formation comprise chelators that can sequester iron, and thereby retard bacterial growth, including aminopolycarboxylates such as ethylenediamine tetraacetic acid (EDTA) or higher homologues such as diethylenetriamine pentaacetic acid (DTPA). Deodorant actives other than astringent metal antiperspirant salts are commonly employed at a concentration of from 0.1 to 5, and particularly 0.1 to 2 % by weight of the topical skin lightening composition of the second aspect of the invention, including all ranges sunsumed therein.

When making the topical skin lightening composition of the second aspect of the invention, the desired ingredients are mixed in no particular order and usually at temperatures from 70 to 80 °C and under atmospheric pressure.

The packaging for the composition of this invention can be a patch, bottle, tube, roll-ball applicator, propellant driven aerosol device, squeeze container or lidded jar.

In a third aspect of the invention, a method of skin lightening is provided, the method of skin lightening comprising the step of applying to skin the topical skin lightening composition of the second aspect of the invention.

The skin lightening benefit may be any one of the benefits selected from the group consisting of treating melasma, treating post-inflammatory hyperpigmentation, treating age spots, treating dark spots and treating uneven skin tone.

### Examples

### Methods:

### (a) General method for the synthesis of compounds of the first aspect of the invention

Compounds were prepared following the synthetic chemistry route set out below.

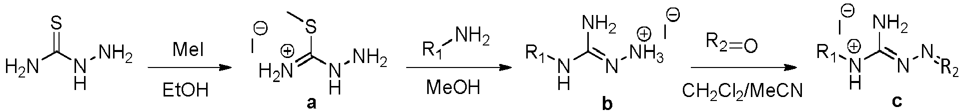

### Synthesis of compound a.

To a suspension of hydrazine carbothioamide (50 mmol) in EtOH (20 mL), methyl iodide (50 mmol, 1 eq) was added. The reaction mixture was heated under microwave irradiation for 1 h at 100°C then allowed to cool for 2 h to permit full precipitation. The compound was filtered, washed with Et2O and dried to afford the desired product as a solid (46.7 g, 80%, CAS: 35600-34-1). Analysis:
¹H NMR: (400 MHz, *d₆*DMSO) δ: 10.40 (1H, br. s), 8.80 (2H, br. s), 5.20 (2H, br. s), 2.60 (3H, s); ¹³C NMR: (400 MHz, *d₆*DMSO) δ: 168.11(C), 13.82 (CH₃).
IR: 3336.0 (w), 3292.1 (w), 3194.6 (br), 3134.5 (br), 1643.1 (s), 1608.2 (s), 1449.4 (s), 1382.9 (m), 13010.3 (m), 1163.8 (m), 961.0 (s).

### Synthesis of compounds of type b.

To a suspension of the suitable amine (R₁-NH2) (0.60 mmol) in MeOH (7 mL), compound a (0.60 mmol) was added. The reaction mixture was stirred at room temperature for 1 h. The volatiles were evaporated and the solid was triturated with a mixture of Et2O and EtOH. The solid was filtered, washed with Et2O and dried under vacuum.

### Synthesis of the final compounds of type c

To a suspension of the suitable active carbonyl compound (R₂=O; 0.24 mmol) in CH2Cl2 (1.5 mL) and MeCN (1.5 mL), compound b was added. The mixture was stirred at 120°C in a microwave oven for 1 h. A solid crashed out from the reaction mixture, was filtered and washed with MeCN and Et2O affording the desired compound as a solid.

### (b) Characterisation of compounds of the first aspect of the invention

The results of characterisation using proton and C13 nuclear magnetic resonance (NMR) and high resolution mass spectroscopy (electrospray ionization) (HRMS (ESI)) of compounds of the first aspect of the invention are shown below.

### NC-0498

### (E)-1-amino-N-(4-hydroxybenzyl)-1-{(2E)-[4-(pyridin-4-yl)benzylidene]hydrazinylidene}methanaminium iodide

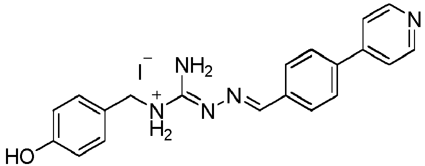

¹H NMR (400 MHz, *d₆*-DMSO) δ: 9.48 (s br, 1H), 8.79 (d, *J* = 6.1 Hz, 2H) 8.29 (s, 1H), 8.18-8.12 (m, 2H), 8.09-8.04 (m, *J* = 8.0 Hz, 3H), 8.00-7.96 (d, *J* = 8.0 Hz, 2H), 7.19-7.15 (d, *J* = 8.5 Hz, 2H), 6.78-6.74 (d, *J* = 8.5 Hz, 2H), 4.62-4.60 (d, *J* = 6.15 Hz, 2H); ¹³C NMR (100 MHz, *d₆*-DMSO) δ: 157.77, 157.02, 154.12, 149.85, 146.93, 137.47, 135.33, 130.61, 128.76, 127.76, 127.14, 122.48, 115.40, 43.79; HRMS (ESI) calculated for [C₂₀H₂₀O₁N₅]⁺, [M - HI + H]⁺ 346.1668, found 346.1667, Δ = 2.6 ppm.

### NC-510

### (E)-1-amino-1-[(2E)-(1-benzothiophen-3-ylmethylidene)hydrazinylidene]-N-[4-(methoxycarbonyl)benzyl]methanaminium iodide

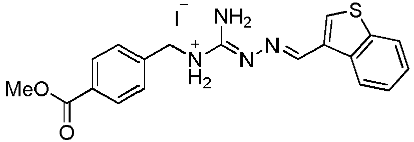

¹H NMR (400 MHz, *d₆*-DMSO) δ: 8.40 (s, 1H), 8.13-8.04 (m, 2H), 7.95 (d, *J* = 8.2 Hz, 2H), 7.55-7.50 (d, *J* = 8.2 Hz, 2H), 7.49-7.41 (m, *J* = 8.0 Hz, 2H), 5.72 (d, *J* = 6.0 Hz, 2H), 3.84 (s, 3H); ¹³C NMR (100 MHz, *d₆*-DMSO) δ: 166.07, 154.36, 142.97, 140.04, 135.32, 134.11, 129.90, 129.53, 129.29, 128.80, 127.25, 125.50, 125.39, 124.93, 123.06, 52.25, 43.92; HRMS (ESI) calculated for [C₁₉H₁₉O₂N₄S₁]⁺, [M - HI + H]⁺ 367.1223, found 367.1218, Δ = -1.46 ppm.

### NC-511

### (E)-1-amino-N-[4-(methoxycarbonyl)benzyl]-1-[(2E)-(quinolin-2-ylmethylidene)hydrazinylidene]methanaminium iodide

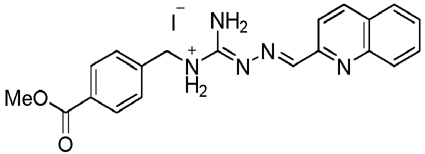

¹H NMR (400 MHz, *d₆*-DMSO) δ: 8.47-8.44 (m, 2H), 8.35 (s br, 1H), 8.05-8.00 (m, 1H) 7.96-7.92 (m, *J₁* = 8.3 Hz, *J₂* = 1.5 Hz, 2H), 7.82-7.78 (td, *J₁* = 7.0 Hz, *J₂* = 1.5 Hz, 1H), 7.67-7.64 (m, *J₁* = 8.0 Hz, *J₂* = 1.1 Hz, 1H), 7.61-7.60 (d, *J* = 8.35 Hz, 2H), 7.51-7.50 (d, *J* = 8.35 Hz, 2H), 4.73 (d, *J* = 6.4 Hz, 2H); ¹³C NMR (100 MHz, *d₆*-DMSO) δ: 166.08, 165.97, 153.00, 142.76, 139.42, 130.34, 129.65, 129.58, 129.46, 129.22, 128.85, 128.23, 128.12, 127.74, 127.27, 118.34, 52.38, 40.20; HRMS (ESI) calculated for [C₂₀H₂₀O₂N₅]⁺, [M - HI + H]⁺ 362.1617, found 362.1628, Δ = 3.0 ppm.

### NC-515

### (E)-1-amino-1-[(2E)-(1-benzothiophen-3-ylmethylidene)hydrazinylidene]-N-(3,4-dichlorobenzyl)methanaminium iodide

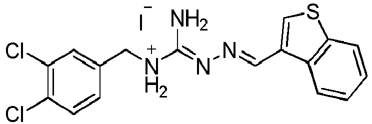

¹H NMR (400 MHz, *d₆*-DMSO) δ: 8.85-8.69 (m br, 2H), 8.59 (d, *J* = 7.7 Hz, 1H), 8.41 (s, 1H), 8.41 (s br, 2H), 8.01 (d, *J* = 7.8 Hz, 1H), 7.70 (s, 1H), 7.60 (d, *J* = 7.9 Hz, 1H), 7.47-7.41 (m, *J₁* = 7.9 Hz, 4H), 4.47 (d, *J* = 6.3 Hz, 2H); ¹³C NMR (100 MHz, *d₆*-DMSO) δ: 154.66, 140.35, 139.06, 135.73, 135.57, 134.25, 131.58, 131.51, 131.43, 131.09, 130.50, 129.96, 128.06, 125.79, 125.25, 123.34, 43.41; HRMS (ESI) calculated for [C₁₇H₁₅Cl₂N₄S₁]⁺, [M - HI + H]⁺ 377.0394, found 377.0407, Δ = 3.5 ppm.

### NC-517

### (E)-1-amino-1-[(2E)-(1-benzothiophen-3-ylmethylidene)hydrazinylidene]-N-(4-fluorobenzyl)methanaminium iodide

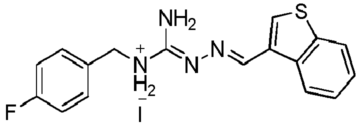

¹H NMR (400 MHz, *d₆*-DMSO) δ: 8.79-8.59 (m, 2H), 8.41 (s, 1H), 8.24 (s br, 1H), 8.01 (d, *J* = 7.7 Hz, 1H), 7.43 (m, 6H), 4.73 (d, *J* = 6.4 Hz, 2H); ¹³C NMR (100 MHz, *d₆*-DMSO) δ: 154.73, 144.05, 140.36, 136.84, 135.74, 134.22, 133.52, 132.52, 130.27, 129.58, 128.90, 125.80, 125.28, 123.35, 43.89; HRMS (ESI) calculated for [C₁₇H₁₅F₁N₄S₁]⁺, [M - HI + H]⁺ 327.1080, found 327.1086, Δ = 1.2 ppm.

### NC-519

### (E)-1-amino-1-[(2E)-(1-benzofuran-2-ylmethylidene)hydrazinylidene]-N-benzylmethanaminium iodide

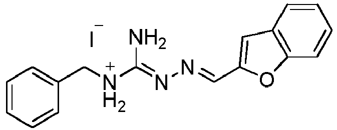

¹H NMR (400 MHz, *d₆*-DMSO) δ: 8.35 (s br, 1H), 8.18 (s br, 2H), 8.79-8.59 (m, 2H), 7.73-7.72 (d, *J* = 7.7 Hz, 1H), 7.61-7.59 (d, *J* = 7.7 Hz, 1H), 7.49 (s, 1H), 7.42-7.37 (m, 6H), 7.35-7.28 (m, 2H), 4.61 (d, *J* = 6.4 Hz, 2H); ¹³C NMR (100 MHz, *d₆*-DMSO) δ: 154.88, 154.13, 150.39, 137.17, 128.64, 128.51, 127.73, 127.59, 127.27, 127.59, 127.27, 126.63, 123.78, 122.26, 44.09; HRMS (ESI) calculated for [C₁₇H₁₇O₁N₄]⁺, [M - HI + H]⁺ 293.1402, found 293.1395, Δ = -2.4 ppm.

### NC-521

### (E)-1-amino-1-[(2E)-(1-benzofuran-2-ylmethylidene)hydrazinylidene]-N-[4-(methoxycarbonyl)benzyl]methanaminium iodide

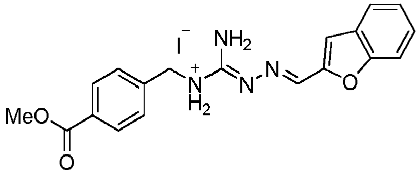

¹H NMR (400 MHz, *d₆*-DMSO) δ: 8.88 (s br, 1H), 8.39 (s br, 2H), 7.96-7.95 (d, *J* = 8.2 Hz, 2H), 7.72-7.70 (d, *J* = 7.7 Hz, 1H), 7.65-7.63 (d, *J* = 8.2 Hz, 1H), 7.60-7.58 (d, *J* = 8.2 Hz, 1H), 7.50-7.49 (d, *J* = 7.0 Hz, 2H), 7.41-7.38 (m, *J* = 7.5 Hz, 1H), 7.30-7.27 (m, *J* = 7.5 Hz, 1H), 4.73-4.72 (d, *J* = 5.8 Hz, 2H), 3.83 (s, 3H); ¹³C NMR (100 MHz, *d₆*-DMSO) δ: 166.10, 154.90, 154.31, 150.40, 142.91, 139.55, 129.55, 129.35, 128.79, 127.75, 127.51, 126.64, 123.78, 122.27, 111.52, 52.26, 43.86; HRMS (ESI) calculated for [C₁₇H₁₇O₁N₄]⁺, [M - HI + H]⁺ 293.1402, found 293.1395, Δ= -2.4 ppm.

### NC-522

### (E)-1-amino-1-[(2E)-(1-benzothiophen-2-ylmethylidene)hydrazinylidene]-N-[4-(methoxycarbonyl)benzyl]methanaminium iodide

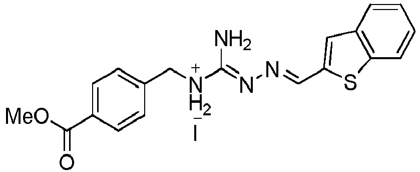

¹H NMR (400 MHz, *d₆*-DMSO) δ: 9.19-8.74 (m, 1H), 8.68 (s br, 1H), 8.22 (s br, 2H), 7.95 (m, *J* = 6.6 Hz, 3H), 7.86 (m, 2H), 7.50 (d, *J* = 8.0 Hz, 2H), 7.40 (m, *J* = 6.6 Hz, 2H), 4.76 (d, *J* = 6.5 Hz, 2H), 3.82 (s, 3H); ¹³C NMR (100 MHz, *d₆*-DMSO) δ: 166.45, 154.65, 143.31, 143.06, 140.19, 139.48, 138.53, 129.84, 129.38, 129.14, 127.73, 126.75, 125.36, 125.04, 123.09, 52.59, 44.29; HRMS (ESI) calculated for [C₁₉H₁₉O₂N₄S₁]⁺, [M - HI + H]⁺ 367.1229, found 367.1236, Δ = 1.9 ppm.

### NC-525

### (E)-1-amino-1-[(2E)-(1-benzothiophen-3-ylmethylidene)hydrazinylidene]-N-[3-(methoxycarbonyl)benzyl]methanaminium iodide

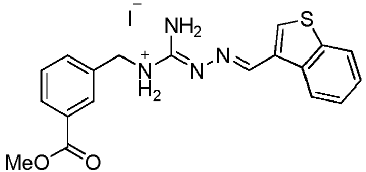

¹H NMR (400 MHz, *d₆*-DMSO) δ: 8.77 (m, 1H), 8.69-8.50 (m, 2H), 8.41 (s, 1H), 8.29-8.13 (s br, 2H), 8.11-7.92 (m,2H), 7.88 (d, *J* = 7.7 Hz, 1H),7.71 (d, *J* = 7.7 Hz, 1H), 7.50 (m, 3H), 4.78 (d, *J* = 6.5 Hz, 2H), 3.82 (s, 3H); ¹³C NMR (100 MHz, d₆-DMSO) δ: 166.53, 154.73, 144.17, 140.38, 138.63, 135.72, 134.33, 132.56, 130.31, 130.28, 129.49, 128.69, 128.28, 125.82, 125.75, 125.31, 123.37, 52.67, 44.14; HRMS (ESI) calculated for [C₁₉H₁₉O₂N₄S₁]⁺, [M - HI + H]⁺ 367.1229, found 367.1240, Δ = 3.0 ppm.

### NC-528

### (E)-1-amino-1-[(2E)-(1-benzothiophen-3-ylmethylidene)hydrazinylidene]-N-(4-methoxybenzyl)methanaminium iodide

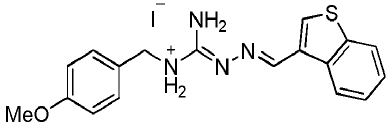

¹H NMR (400 MHz, *d₆*-DMSO) δ: 8.55-8.50 (m, 2H), 8.38 (s, 1H), 8.07-8.05 (m, 1H), 7.99 (s br, 1H), 7.49-7.46 (m, *J₁* = 5.3 Hz, *J₂* = 1.6 Hz, 2H), 7.33-7.31 (d, *J* = 8.7 Hz, 2H), 6.97-6.95 (d, *J* = 8.7 Hz, 2H), 4.53-4.52 (d, *J* = 6.1 Hz, 2H), 3.73 (s, 3H); ¹³C NMR (100 MHz, *d₆*-DMSO) δ: 158.84, 154.06, 143.85, 140.051, 135.34, 130.57, 129.93, 129.03, 128.724, 125.50, 125.39, 124.88, 123.08, 114.09, 55.29, 43.73; HRMS (ESI) calculated for [C₁₈H₁₉ON₄S₁]⁺, [M - HI + H]⁺ 339.1286, found 339.1280, Δ = 1.8 ppm.

### NC-531

### (E)-1-amino-1-[(2E)-(1-benzofuran-2-ylmethylidene)hydrazinylidene]-N-(4-methoxybenzyl)methanaminium iodide

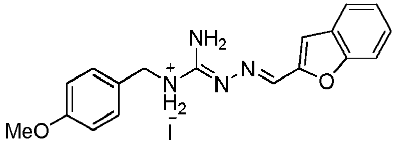

¹H NMR (400 MHz, *d₆*-DMSO) δ: 8.78-8.70 (s br, 1H), 8.23 (m br, 2H), 7.69 (d, *J* = 7.7 Hz, 1H), 7.58 (d, *J* = 7.7 Hz, 1H), 7.50 (s, 1H), 7.37-7.20 (m, *J₁* = 8.2 Hz, 4H), 6.91 (d, *J* = 8.2 Hz, 2H), 4.57 (d, *J* = 6.4 Hz, 2H), 3.74 (s, 3H); ¹³C NMR (100 MHz, *d₆*-DMSO) δ: 259.16, 155.23, 154.44, 150.78, 131.01, 129.42, 128.10, 126.92, 126.41, 124.09, 122.57, 114.36, 111.85, 111.45, 55.62, 44.07; HRMS (ESI) calculated for [C₁₈H₁₉O₂N₄]⁺, [M - HI + H]⁺ 323.1508, found 323.1518, Δ = 3.1 ppm.

### NC-0536

### (E)-1-amino-N-(4-fluorobenzyl)-1-{(2Z)-[6-methyl-2,3,4,9-tetrahydro-1H-carbazol-1-ylidene]hydrazinylidene}methanaminium iodide

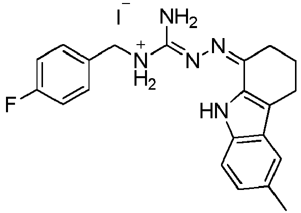

¹H NMR (400 MHz, *d₆*-DMSO) δ: 7.43 (m, 4H), 7.27 (s, 1H), 7.24 (d, *J* = 8.3 Hz, 1H), 6.99 (d, *J* = 8.3 Hz, 1H), 4.63 (d, *J* = 6.6, 2H), 2.75 (t, *J* = 6.3 Hz, 2H), 2.68 (t, *J* = 6.3 Hz, 2H), 2.35 (s, 3H), 1.97 (quin, *J* = 6.3 Hz, 2H); ¹³C NMR (100 MHz, *d₆*-DMSO) δ: 145.77, 136.87, 135.91, 132.54, 130.95, 129.53, 128.74, 127.86, 126.87, 125.96, 120.70, 119.29, 112.96, 111.51, 38.57, 25.74, 23.35, 21.52, 20.57; HRMS (ESI) calculated for [C₂₁H₂₃N₅F₁]⁺, [M - HI + H]⁺ 364.1937, found 364.1939, Δ = 0.4 ppm.

### NC-0537

### (E)-1-amino-N-(2,6-dichlorobenzyl)-1-{(2Z)-[6-methyl-2,3,4,9-tetrahydro-1H-carbazol-1-ylidene]hydrazinylidene}methanaminium iodide

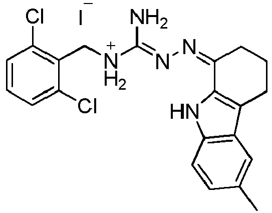

¹H NMR (400 MHz, *d₆*-DMSO) δ: 7.65 (m, 1H), 7.49 (m, 2H), 7.31 (s, 1H), 7.18 (d, *J* = 8.4, 1H), 6.98 (d, *J* = 8.4, 1H), 6.47 (s br., 1H), 4.66 (d, *J* = 6.4 Hz, 2H), 2.73 (m, 4H), 2.34 (s, 3H), 1.98 (m, 2H); ¹³C NMR (100 MHz, *d₆*-DMSO) δ: 164.60, 157.71, 145.88, 135.93, 130.93, 129.35, 128.00, 127.85, 126.86, 125.96, 119.28, 118.94, 111.51, 87.54, 43.73, 25.83, 23.38, 21.57, 20.59; HRMS (ESI) calculated for [C₂₁H₂₂N₅Cl₂]⁺, [M - HI + H]⁺414.1247, found 414.1244, Δ = - 0.6 ppm.

### (c) Assay of the inhibition of pigment production in B16 mouse melanoma cells by compounds of the first aspect of the invention

B16 mouse melanoma cells are widely available for example by purchase from the American Type Culture Collection (ATCC) and are routinely used by those skilled in the art to assay pigment production. The cells make and secrete melanin, a dark pigment, into the medium as the cells approach confluence. Methods for general cell culture may be easily accessed in text books and on-line (eg General Techniques of Cell Culture; M.A. Harrison and I.F. Rae Authors; Cambridge University Press ISBN 052157496X)

B16 mouse melanoma cells were maintained in cell culture and used for tests in antibiotic free Lonza Biowhittaker (BE12-662F) media (50ml fetal bovine serum and 5 ml I-glutamine (Sigma G7513) per 500 ml bottle).

The cells were maintained and grown at 37°C in an atmosphere of 5 % CO2. Trypsin/ethylene diamine tetraacetic acid (EDTA; Sigma T3924) and Dulbeccos Phosphate Buffered Saline (DPBS; Sigma D8537) were used to passage the cells as they approached ≥80 % confluence, diluting the cells by one tenth for maintenance culture.

For the pigment reduction assays, 48 well plates were seeded with 2.5 x 10⁴ cells per well in phenol red-free, antibiotic-free medium (Sigma D1145) supplemented with foetal bovine serum and glutamine. Care was taken to make sure less adherent cells were not lost during the seeding process.

The plates were incubated overnight at 37°C/5 % CO₂ atmosphere. The media was removed from the plates one-by-one and fresh media containing the test compound or control additions was added to each well. Compounds were prepared as stock solutions at 1000 fold higher concentration than that used for assay in dimethylsulphoxide (DMSO). Control media additions included 0.1 % DMSO, media alone and positive control(s) known to inhibit pigment production such as ketoconazole or 4-ethylresorcinol. The cells were incubated at 37°C/5 % CO₂ atmosphere for a further 3 days. Cultures were assessed for melanin secretion into the medium and, as controls for toxicity of the added ingredients, for protein level and metabolic activity. A reduction in metabolic activity or protein levels is indicative of cell toxicity. The most desirable compounds therefore in the assay are those that show melanin production inhibition without loss of metabolic function or reduction in protein levels.

### Assessment of melanin concentration

Media was removed from the cell plates and put into storage plates. The plates were sealed until assayed later in the day. Melanin was measured by determining optical density at 450 nm and making a comparison against melanin standards (Sigma M8631) made up anew on each day of testing. The standards used were 1000 µg/ml; 500 µg/ml; 250 µg/ml; 125 µg/ml; 62.5 µg/ml; 31.25 µg/ml; and 15.625 µg/ml.

### WST-1 cell viability assay

The stable tetrazolium salt WST-1 (Water Soluble Tetrazolium Salt -1) is cleaved to a soluble formazan by a complex cellular mechanism that occurs primarily at the cell surface. This bioreduction is largely dependent on the glycolytic production of nicotinamide adenine dinucleotide phosphate (NADPH) in viable cells. Therefore, the amount of formazan dye formed directly correlates to the number of metabolically active cells in the culture. Without delay, 200 µl fresh, warmed, phenol red free media with WST-1 (used as per manufacturer's instruction) was added to each well and plates incubated at 37°C/5 % CO₂ atmosphere for 1 hour. Media was removed from plates into storage plates taking care to remove as much as possible, but not to scratch the cell layer. The formazan dye formed was measured at 450 nm using an enzyme-linked immunosorbent assay (ELISA) reader. The measured absorbance directly correlates to the number of viable cells in the culture well.

### Protein determination

Without delay, 100 µl of RIPA buffer (Sigma R0278) with protease inhibitor (Sigma S8830- use as per Sigma instructions) was added to the wells and the plates frozen at -20°C until the protein assessment was carried out. For example Pierce BCA Protein Assay (Thermo Scientific # 23225) can be used as per manufacturer's instructions with 10 µl of sample for each replicate.

### Results

Melanin production for each compound tested is shown in Table 1. Control and test melanin values were calculated as the average of results from 4 wells, corrected for protein levels in the cultures. The percent reduction in melanin production was calculated for each compound concentration, setting the melanin production of control incubations (vehicle alone) at 100%. Compound concentrations causing > 20% loss of WST activity are considered toxic and not considered to be effective melanin inhibitors. None of the compounds shown in Tables 1 and 2 were toxic. A compound causing > 10% reduction of melanin production into the medium was considered to be an inhibitor.

**Table 1: B16 mouse melanoma cells assay results for compounds of the first aspect of the invention**

| | | | **% of melanin inhibition in B16 mouse melanoma cells assay compared to control, normalised to protein levels** | |
|---|---|---|---|---|
| **NC-number** | **R₁** | **R₂** | **5 µM** | **1 µM** |
| NC-0498 | 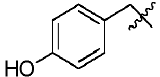 | 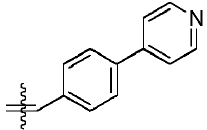 | 33 | 0 |
| NC-0510 | 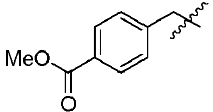 | 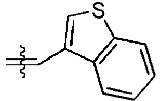 | 29 | 17 |
| NC-0511 | 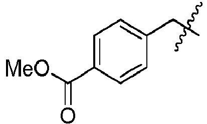 | 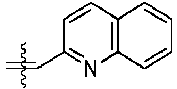 | 100 | 54 |
| NC-0515 | 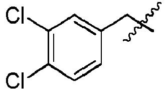 | 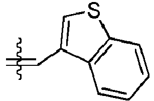 | 54 | 0 |
| NC-0517 | 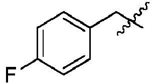 | 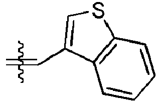 | 35 | 32 |
| NC-0519 | 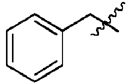 | 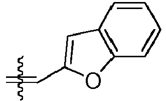 | 71 | 71 |
| NC-0521 | 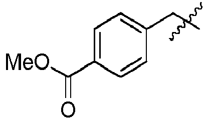 | 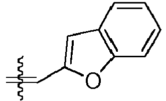 | 64 | 46 |
| NC-0522 | 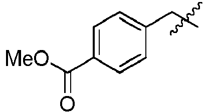 | 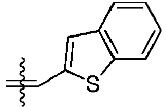 | 53 | 59 |
| NC-0525 | 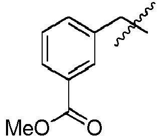 | 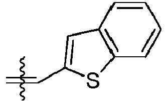 | 100 | 18 |
| NC-0528 | 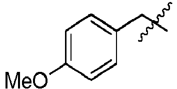 | 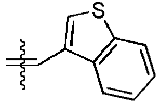 | 73 | 3 |
| NC-0531 | 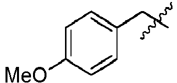 | 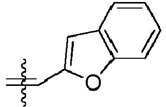 | 39 | 39 |
| NC-0536 | 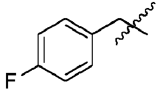 | 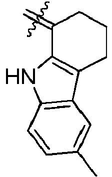 | 8 | 24 |
| NC-0537 | 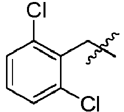 | 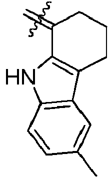 | 37 | 51 |

### (d) Assay of pigment production by Living Skin Equivalents (Melanoderms™) and effect of compounds of the first aspect of the invention

So called reconstructed pigmented 'skin' cultures containing keratinocytes and melanocytes are commercially available and for example can be purchased from MatTek Corp, 200, Homer Avenue, Ashland, MA, USA, under the trade name Melanoderm^{™}. Melanoderms^{™} (catalogue reference MEL-300) containing melanocytes from black donor skin were maintained in EPI-100-NMM113 media following the manufacturer's instructions and using standard methods for the maintenance of cultured mammalian cells under aseptic conditions. The Melanoderm^{™} cultures were treated with test agents for a total of 14 days. During the 14 day culture period the melanocytes in the Melanoderm^{™} cultures synthesise melanin in specialised vesicular structures called melanosomes. The pigmented melanosomes are transferred to the adjacent keratinocytes and the transferred-melanosomes tend to accumulate over the nucleus of the keratinocytes. This process is the same as occurs in skin in vivo and accordingly the Melanoderm^{™} culture is considered to be a very good test 'skin tissue' to mimic the effect of the skin pigmentation process in vivo. A compound that leads to a reduction in melanin production in the Melanoderm^{™} culture over 14 days by 10 % or more is considered to have a positive pigment reducing effect. It is important that the reduction in pigment occurs without substantive loss of cell viability such that the inhibitory effect of the test compound is due to its biological function rather than due to cell toxicity.

Culture media changes and re-dosing of compounds at selected concentrations took place every 2 or 3 days during the test period. Melanoderm^{™} cultures were also treated with control materials including the vehicle (dimethylsulphoxide; DMSO) used to dissolve the test material and a positive control material known to lead to the synthesis of reduced amounts of melanin in the cultures. Ketoconazole and/or 4-ethylresorcinol were used as positive controls. After 14 days culture, transmitted-light microscopy images were captured for every culture using a Leica DM IL microscope at x20 magnification.

The cell viability of the cultures was assessed using the so called WST-1 (Water Soluble Tetrazolium Salt -1) assay purchased from Roche Applied Science and following the manufacturer's instructions. The stable tetrazolium salt WST-1 is cleaved to a soluble formazan dye by a complex cellular mechanism that occurs primarily at the cell surface. This bioreduction is largely dependent on the glycolytic production of NADPH in viable cells. Therefore, the amount of soluble formazan dye formed in the culture medium directly correlates to the number of metabolically active cells in the culture. Cultures were incubated with WST-1 in the medium for 1 hour and production of formazan measured at 450 nm using a spectrophotometer plate reader, eg a BMG Labtech FLUOstar plate reader. Cultures were considered 'viable' if they generated greater than 80 % of the control value of formazan product. Subsequently the culture was washed briefly in phosphate buffer solution (PBS) and total melanin and protein extracted together from each culture using 250 µl Solvable (trademark, Perkin Elmer product 6NE9100) reagent at 60°C overnight as recommended by the manufacturer. The total protein content using a sample of the extracts was determined using the so called BCA (bicinchoninic acid) assay. The BCA assay may be purchased from Pierce Chemical Company and conducted following the manufacturer's instructions. Melanin was determined by measuring the absorbance at 485 nm, for example using a BMG Labtech FLUOstar plate reader. The amount of melanin extracted from each culture was determined by reference to a standard synthetic-melanin curve (Sigma Aldrich Chemical Co; catalogue reference M8631).

### Results

Melanin production for each compound tested is shown in Table 2. Melanin levels in each culture were expressed as values normalised to the amount of protein in the cultures and compared to control vehicle treated cultures. A reduction of greater than 10% in the protein normalised melanin content of the cultures without substantive loss of cell viability, was considered to be a successful and novel pigment reducing effect. This shows that compounds of the first aspect of the invention reduce melanin production in vitro and thereby may be used to beneficially alter human skin colour.

**Table 2: Melanoderm^{™} assay results for compounds of the first aspect of the invention**

| **Inhibition of melanin production (%) in Melanoderms^{™} compared to control by compounds of the first aspect of the invention. Melanin production was normalized to protein levels as described in the methods. Compounds were tested in separate experiments at 5 µM and 10 µM. ND = Not Done** | | |
|---|---|---|
| **NC-Number** | **10 µM** | **5 µM** |
| NC-0498 | 53 | ND |
| NC-0510 | 19 | 18 |
| NC-0511 | 19 | 27 |
| NC-0517 | 20 | 4 |
| NC-0519 | 47 | 40 |
| NC-0521 | ND | 35 |
| NC-0522 | 31 | 34 |
| NC-0531 | 40 | 35 |

## Claims

1. A compound of structure I: 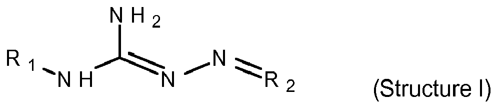 or a salt or a stereoisomer thereof,
wherein R₁ is 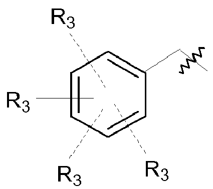 wherein R₃ = F, Cl, OCH₃, COOCH₃, OH or Me;
wherein if R₂ is a benzofuran moiety or 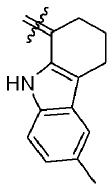 R₁ can be a phenyl group;
wherein R₂ is 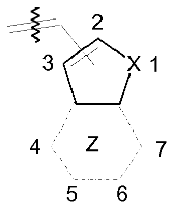 connected via either one of the two carbon atoms numbered 2 or 3;
wherein X1 = O or S;
wherein if Z is not present, the heterocycle containing X1 contains another double bond; wherein Z is a six membered aromatic or non-aromatic heterocyclic, or cycloalkyl or aryl moity, optionally substituted at one or more available carbons numbered 4 to 7 with R₃;
or R₂ is 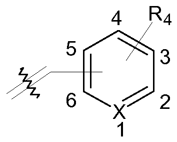 connected via either one of the two carbon atoms numbered 5 or 6;
wherein X1 = C or N;
wherein R₄ is a 6 membered pendant monoheterocyclic ring attached at any available carbon, or a 6 membered fused aromatic ring, fused at any available pair of carbons;
or R₂ is

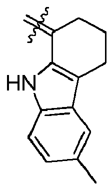

2. A compound according to claim 1 wherein Z is not substituted at one or more available carbons numbered 4 to 7 with R₃.

3. A compound according to claim 1 or claim 2 wherein Z is aromatic.

4. A compound according to any one of the preceding claims wherein R₄ is an aromatic ring.

5. A compound according to any one of the preceding claims wherein R₁ is singly or doubly substituted by R₃.

6. A compound according to claim 1 or claim 5 wherein R₂ is a benzothiophene or a benzofuran moiety.

7. A compound according to claim 1 or claim 5 wherein R₂ is a quinoline moiety.

8. A compound according to any one of the preceding claims wherein the stereoisomer is the E stereoisomer.

9. A compound according to any one of claims 1 to 7 wherein the stereoisomer is the Z stereoisomer.

10. A compound according to any one of the preceding claims which is a salt of structure (I) and wherein the counterion is selected from the group consisting of phosphate, hydrogen monophosphate, hydrogen biphosphate, carbonate, bicarbonate, formate, acetate, nitrate, sulphate, hydrogen sulphate, sulphite, metabisulphite, hydroxide, fluoride, chloride, bromide, iodide, lactate, salicyclate, phytate, lipoate, tannate, gallate, a fatty acid anion and an organic fruit acid anion.

11. A compound according to claim 10 wherein the fatty acid anion is stearate or palmitate.

12. A compound according to claim 10 or claim 11 wherein the organic fruit acid anion is selected from the group consisting of malate, quinate, alpha ketoglutarate, oxalacetate, citrate, pyruvate, fumarate, lactate, succinate, tartrate, acetate, formate, isocitrate, lactoisocitrate, shikimate, oxalate, glycerate citramalate, glycolate, glucuronate, galacturonate, glutamate, aspartate, benzoate, ascorbate, salicylate and sorbate.

13. A compound according to claim 1 selected from the group consisting of (E)-1-amino-N-(4-hydroxybenzyl)-1-{(2E)-[4-(pyridin-4-yl)benzylidene]hydrazinylidene}methanamine (NC-0498), (E)-1-amino-1-[(2E)-(1-benzothiophen-3-ylmethylidene)hydrazinylidene]-N-[4-(methoxycarbonyl)benzyl] methanamine (NC-0510), (E)-1-amino-N-[4-(methoxycarbonyl)benzyl]-1-[(2E)-(quinolin-2-ylmethylidene)hydrazinylidene] methanamine (NC-511), (E)-1-amino-1-[(2E)-(1-benzothiophen-3-ylmethylidene)hydrazinylidene]-N-(3,4-dichlorobenzyl) methanamine (NC-0515), (E)-1-amino-1-[(2E)-(1-benzothiophen-3-ylmethylidene)hydrazinylidene]-N-(4-fluorobenzyl) methanamine (NC-0517), (E)-1-amino-1-[(2E)-(1-benzofuran-2-ylmethylidene)hydrazinylidene]-N-benzylmethanaminium iodide (NC-519), (E)-1-amino-1-[(2E)-(1-benzofuran-2-ylmethylidene)hydrazinylidene]-N-[4-(methoxycarbonyl)benzyl] methanamine (NC-0521), (E)-1-amino-1-[(2E)-(1-benzothiophen-2-ylmethylidene)hydrazinylidene]-N-[4-(methoxycarbonyl)benzyl] methanamine (NC-0522), (E)-1-amino-1-[(2E)-(1-benzothiophen-3-ylmethylidene)hydrazinylidene]-N-[3-(methoxycarbonyl)benzyl] methanamine (NC-0525), (E)-1-amino-1-[(2E)-(1-benzothiophen-3-ylmethylidene)hydrazinylidene]-N-(4-methoxybenzyl)methanaminium iodide (NC-0528), (E)-1-amino-1-[(2E)-(1-benzofuran-2-ylmethylidene)hydrazinylidene]-N-(4-methoxybenzyl) methanamine (NC-0531), (E)-1-amino-N-(4-fluorobenzyl)-1-{(2Z)-[6-methyl-2,3,4,9-tetrahydro-1H-carbazol-1-ylidene]hydrazinylidenel methanamine (NC-0536), (E)-1-amino-N-(2,6-dichlorobenzyl)-1-{(2Z)-[6-methyl-2,3,4,9-tetrahydro-1H-carbazol-1-ylidene]hydrazinylidenel methanamine (NC-0537), or a salt or stereoisomer thereof.

14. A compound according to claim 13 selected from the group consisting of (E)-1-amino-1-[(2E)-(1-benzothiophen-3-ylmethylidene)hydrazinylidene]-N-[4-(methoxycarbonyl)benzyl] methanamine (NC-0510), (E)-1-amino-N-[4-(methoxycarbonyl)benzyl]-1-[(2E)-(quinolin-2-ylmethylidene)hydrazinylidene] methanamine (NC-0511), (E)-1-amino-1-[(2E)-(1-benzothiophen-3-ylmethylidene)hydrazinylidene]-N-(4-fluorobenzyl)methanaminium iodide (NC-0517), (E)-1-amino-1-[(2E)-(1-benzofuran-2-ylmethylidene)hydrazinylidene]-N-[4-(methoxycarbonyl)benzyl] methanamine (NC-521), (E)-1-amino-1-[(2E)-(1-benzothiophen-2-ylmethylidene)hydrazinylidene]-N-[4-(methoxycarbonyl)benzyl] methanamine (NC-0522), (E)-1-amino-1-[(2E)-(1-benzofuran-2-ylmethylidene)hydrazinylidene]-N-(4-methoxybenzyl) methanamine (NC-0531), or a salt or stereoisomer thereof.

15. A compound according to claim 14 selected from the group consisting of (E)-1-amino-N-[4-(methoxycarbonyl)benzyl]-1-[(2E)-(quinolin-2-ylmethylidene)hydrazinylidene] methanamine (NC-0511), (E)-1-amino-1-[(2E)-(1-benzofuran-2-ylmethylidene)hydrazinylidene]-N-[4-(methoxycarbonyl)benzyl] methanamine (NC-0521), (E)-1-amino-1-[(2E)-(1-benzothiophen-2-ylmethylidene)hydrazinylidene]-N-[4-(methoxycarbonyl)benzyl] methanamine (NC-0522), or a salt or stereoisomer thereof.

16. A topical skin lightening composition comprising one or more compounds of any one of the preceding claims and a dermatologically acceptable carrier.

17. A topical skin lightening composition according to claim 16, the composition comprising 0.001-5, preferably 0.01-2, most preferably 0.1-1 % w/w of one or more compounds of any one of claims 1 to 15.

18. A topical skin lightening composition according to claim 16 or claim 17, wherein the topical skin lightening composition is in the form of an emulsion.

19. A topical skin lightening composition according to any one of claims 16 to 18, additionally comprising at least one sunscreen compound.

20. A topical skin lightening composition according to claim 19 wherein the sunscreen compound is organic.

21. A topical skin lightening composition according to claim 19 wherein the sunscreen compound is inorganic.

22. A topical skin lightening composition according to any one of claims 16 to 21 comprising at least one additional skin lightening compound not falling within structure (I), or a salt or a stereoisomer thereof.

23. A topical skin lightening composition according to claim 22 wherein the at least one additional skin lightening compound is selected from the group consisting of niacinamide, a resorcinol, koijic acid, retinol, retinyl esters including retinyl palmitate, 12-hydroxystearic acid, lactic acid, ascorbic acid, glabradin, dioic acids including octadecenedioic acid and azeleic acid, resveratrol, ascorbyl phosphate, ascorbyl palmitate, acetyl glucosamine, calcium pantothenate, alpha arbutin, climbazole, pitera extract, soybean extract, undecylenoyl phenylalanine, aqueous extract of bearberry and mixtures thereof.

24. A topical skin lightening composition according to claim 23 wherein the resorcinol is selected from the group consisting of 4-ethyl resorcinol, 4-hexyl resorcinol and phenylethyl resorcinol.

25. A topical skin lightening composition according to any one of claims 22 to 24, the composition comprising 0.0001-10, preferably 0.01-2, most preferably 0.1-1 % w/w of the at least one additional skin lightening compound.

26. A topical skin lightening composition according to any one of claims 16 to 25 additionally comprising an active selected from the list consisting of an antiperspirant active, an organic sunscreen, and an inorganic sunscreen.

27. A method of skin lightening comprising the step of applying to skin a topical skin lightening composition of claims 16 to 26.

28. A method according to claim 27 wherein skin lightening is any one of the benefits selected from the group consisting of treating melasma, treating post-inflammatory hyperpigmentation, treating age spots, treating dark spots and treating uneven skin tone.
